# DEMANDE DE BREVET EUROPEEN

(11) **EP 4 000 666 A1**
(43) Date de publication de la demande: **25.05.2022**
(21) Numéro de dépôt: 21209371.0
(22) Date de dépôt: 19.11.2021
(51) Int. Cl.: A61M 3/02

(54) **MACHINE POUR METTRE EN OEUVRE LE LAVAGE DU COLON D'UN PATIENT**

(30) Priorité: 23.11.2020 FR 2012002
(71) Demandeur: Horizon Medical, 14260 Aunay sur Odon (FR)
(72) Inventeur: L'HIRONDEL, Christian, VERSON (FR); DURAND, Jean-Paul, AUNAY SUR ODON (FR)
(74) Mandataire: Cabinet Le Guen Maillet

(57) **Abrégé**

La présente invention se rapporte à une machine (100) conçue pour mettre en œuvre le lavage du colon d'un patient, comprenant, un divan (Dv) incluant un bassin de lavage (Bl), un réservoir d'eau de lavage (Rl) disposé plus haut que le bassin de lavage (Bl), dans la position d'utilisation de ladite machine, une canalisation (Clt) d'alimentation en eau raccordée au réservoir d'eau de lavage (Rl), une canule rectale (Cn) raccordée au réservoir d'eau de lavage (Rl), par l'intermédiaire d'une canalisation d'eau de lavage (Clt3). Selon l'invention, un débitmètre (Db) est raccordé sur la canalisation d'eau de lavage (Clt3) qui raccorde le réservoir d'eau de lavage (Rl) avec la canule rectale (Cn).

L'information de débit que peut fournir ce débitmètre est utilisée pour vérifier que de l'eau de lavage circule en continu dans le colon du patient.

## Description

La présente invention concerne une machine pour mettre en œuvre le lavage du colon d'un patient.

On connaît à la lecture du brevet CN-U-206183733, un dispositif de lavage de l'intestin. Il comprend un corps de table d'hydrothérapie comprenant un dossier sur lequel peut s'allonger le patient, un bassin, un réservoir, une unité d'alimentation en eau chaude du réservoir, un capteur de température de l'eau et un système de contrôle de la température de l'eau pénétrant dans le réservoir, un élément de stérilisation de l'eau aux ultraviolets, un système d'alimentation en liquide relié au capteur de température du liquide, ainsi qu'un mécanisme de chasse d'eau raccordé au bassin. La plage de température de l'eau est comprise entre 37 °C et 39 °C. Il est à noter que ce dispositif de lavage a été initialement conçu et développé pour une utilisation dans les spas et que presque toutes les fonctions sont mises en œuvre manuellement et certains réglages sont réalisés par le patient durant la procédure de lavage.

Partant de ce constat, le demandeur a cherché à concevoir une machine pour mettre en œuvre le lavage du colon d'un patient qui puisse être plus sûr.

A cet effet, est proposée une machine conçue pour mettre en œuvre le lavage du colon d'un patient, comprenant un divan incluant un bassin de lavage, un réservoir d'eau de lavage disposé plus haut que le bassin de lavage, dans la position d'utilisation de ladite machine, une canalisation d'alimentation en eau raccordée au réservoir d'eau de lavage, une canule rectale raccordée au réservoir d'eau de lavage, par l'intermédiaire d'une canalisation d'eau de lavage, où un débitmètre est raccordé sur la canalisation d'eau de lavage.

Selon l'invention, une électrovanne est raccordée sur la canalisation d'eau de lavage qui raccorde le réservoir d'eau de lavage avec la canule rectale, la machine comprenant une armoire de commande incluant, un calculateur, un programme mis en œuvre dans le calculateur, l'électrovanne et le débitmètre étant raccordés au calculateur et le programme contient des instructions pour couper le débit d'eau circulant dans l'électrovanne, quand le débit d'eau circulant dans le débitmètre devient nul, et activer une alarme visuelle ou sonore.

L'information de débit que peut fournir ce débitmètre peut être utilisée pour vérifier que de l'eau de lavage circule en continu dans le colon du patient et il est également possible de couper l'écoulement de l'eau si son débit devient trop faible. Ainsi, le praticien qui utilise la machine est averti que l'irrigation du colon du patient n'est plus active. Il peut ainsi intervenir auprès du patient, puis relancer le lavement du colon du patient.

Avantageusement, la machine comprend un capteur thermique monté dans le réservoir d'eau de lavage, le capteur thermique étant raccordés au calculateur et le programme contient des instructions pour couper le débit d'eau circulant dans l'électrovanne, quand la température mesurée par le capteur thermique est supérieure à 39°C, et activer une alarme visuelle ou sonore.

L'information que peut fournir ce capteur thermique peut être utilisée pour mesurer la température de l'eau de lavage amenée à circuler dans le colon du patient et le contrôle de la température de l'eau de lavage garantit la sécurité du patient car l'irrigation du colon du patient n'est plus active. Le praticien qui utilise la machine est averti.

Selon une caractéristique additionnelle de l'invention, un filtre est raccordé sur la canalisation d'alimentation en eau, ledit filtre étant un filtre de microfiltration comprenant une membrane en fibres creuses.

L'eau qui sort de la canule rectale pendant le fonctionnement de la machine est bactériologiquement maîtrisée, c'est à dire ne contenant aucune bactérie.

Selon une caractéristique additionnelle de l'invention, la machine comprend une douchette raccordée à une seconde canalisation d'alimentation en eau, et un second filtre est raccordé sur ladite canalisation d'alimentation en eau, le second filtre étant un filtre de microfiltration comprenant une membrane en fibres creuses.

L'eau qui sort de la douchette pendant le fonctionnement de la machine est bactériologiquement maîtrisée, c'est à dire ne contenant aucune bactérie.

Selon une caractéristique additionnelle de l'invention, le réservoir d'eau de lavage contient un thermoplongeur, un agitateur, qui sont raccordés au calculateur.

Ces composants installés dans le réservoir d'eau de lavage permettent au calculateur de contrôler précisément et en continu la température de l'eau qui est amenée à circuler dans la canule rectale.

Selon une caractéristique additionnelle de l'invention, plusieurs capteurs de niveau d'eau sont montés dans le réservoir d'eau de lavage pour mesurer des niveaux d'eau dans le réservoir d'eau de lavage, lesdits capteurs étant raccordés au calculateur.

Le calculateur peut ainsi surveiller l'évolution du niveau d'eau dans le réservoir d'eau de lavage.

Selon une caractéristique additionnelle de l'invention, l'armoire de commande contient un réservoir de chasse d'eau raccordé à un siphon raccordé sous le bassin de lavage, par l'intermédiaire d'une vanne à commande manuelle.

En actionnant la vanne manuelle, on nettoie le siphon.

Selon une caractéristique additionnelle de l'invention, le bassin de lavage est raccordé à un réseau de collecte des eaux usées, par l'intermédiaire d'une canalisation d'évacuation et dont au moins une portion est transparente, une caméra étant orientée vers la portion transparente pour la voir, la caméra étant reliée à un moniteur vidéo implanté dans l'armoire de commande.

Le praticien peut ainsi évaluer sur le moniteur vidéo, l'évolution de la turbidité des eaux de lavage en provenance du colon du patient.

Les caractéristiques de l'invention mentionnées ci-dessus, ainsi que d'autres, apparaîtront plus clairement à la lecture de la description suivante d'un exemple de réalisation, ladite description étant faite en relation avec les dessins joints, parmi lesquels :
Fig. 1 représente une vue en perspective d'une machine pour mettre en œuvre le lavage du colon d'un patient selon l'invention,
Fig. 2 représente une vue latérale d'une machine pour mettre en œuvre le lavage du colon d'un patient selon l'invention,
Fig. 3 représente une vue de face d'une machine pour mettre en œuvre le lavage du colon d'un patient selon l'invention,

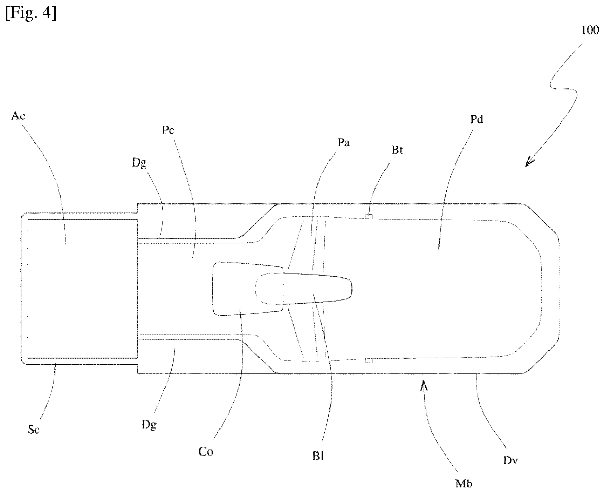

Fig. 4 représente une vue de dessus d'une machine pour mettre en œuvre le lavage du colon d'un patient selon l'invention et,
Fig. 5 représente une vue schématique d'une armoire de commande pour une machine pour mettre en œuvre le lavage du colon d'un patient selon l'invention.

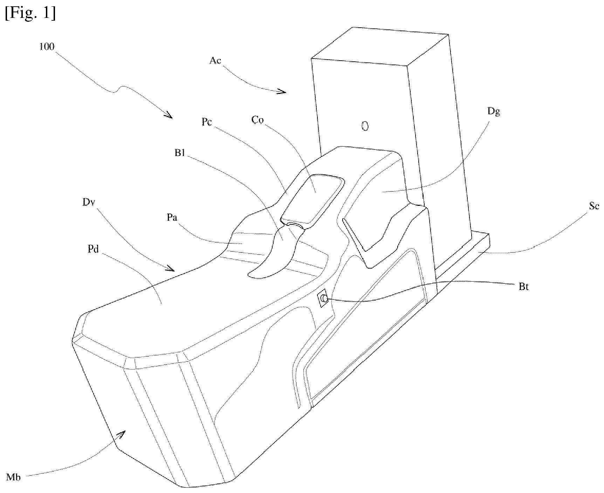

La machine 100 présentée sur la Fig. 1 est conçue pour mettre en œuvre le lavage du colon d'un patient. Elle convient ainsi pour préparer le colon pour un diagnostic médical, notamment une coloscopie ou un colo-scanner, une intervention chirurgicale, traiter une constipation chronique.

Elle est destinée à être utilisée, notamment, dans un hôpital, une clinique.

La machine 100 comprend une embase Mb destinée à reposer sur un sol horizontal et sur lequel est présent un divan Dv d'une conception particulière. Un socle Sc, sur lequel est posé une armoire de commande Ac. L'embase Mb est avantageusement fabriquée en un matériau composite et par thermoformage. L'embase Mb comprend le divan Dv.

Le divan Dv se compose d'une paroi dorsale Pd sensiblement plane dimensionnée pour réceptionner le dos d'un patient allongé, prolongée d'un bord par une paroi arrondie Pa dimensionnée pour réceptionner les fesses du patient, elle-même prolongée de son autre bord par une paroi cuissière Pc destinée à réceptionner la partie antérieure des cuisses du patient. La largeur de la paroi cuissière Pc est réduite dans le prolongement de la paroi arrondie Pa et dans le dégagement ainsi créé latéralement dans le socle Sc de part et d'autre de la paroi cuissière Pc, sont respectivement définis deux dégagements Dg dans lesquels peuvent prendre place les genoux, les mollets et les pieds du patient. La partie basse de ces deux dégagements Dg est définie par deux faces d'appui qui s'étendent latéralement vers l'extérieur et sur lesquelles peuvent prendre appui les deux pieds du patient.

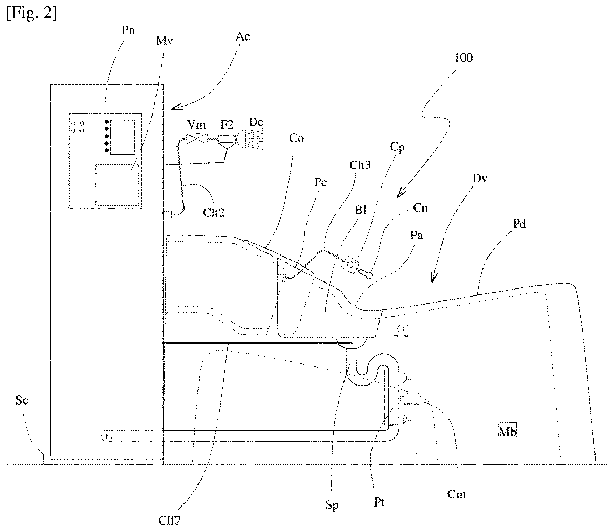

Sur la Fig. 2, l'angle formé entre la paroi dorsale Pd et le plan du sol est de pratiquement 9°. L'angle formé entre cette paroi dorsale Pd et la paroi cuissière Pc est de pratiquement 145°. Ces angles sont donnés avec une tolérance de +/- 5°. Ces plages angulaires procurent un bon confort pour le patient.

Sur les Figs. 1, 2 et 3, un bassin de lavage Bl prend place au travers de la paroi arrondie Pa. Il est conçu pour recueillir et évacuer les eaux de lavage du colon du patient. Il est disposé dans le plan médian longitudinal du divan Dv. Il s'étend en profondeur. Il s'étend longitudinalement également en partie dans la paroi dorsale Pd et dans la paroi cuissière Pc.

Sur la Fig. 1, un capot Co recouvre localement et du côté de la paroi cuissière Cs, le bassin de lavage Bl.

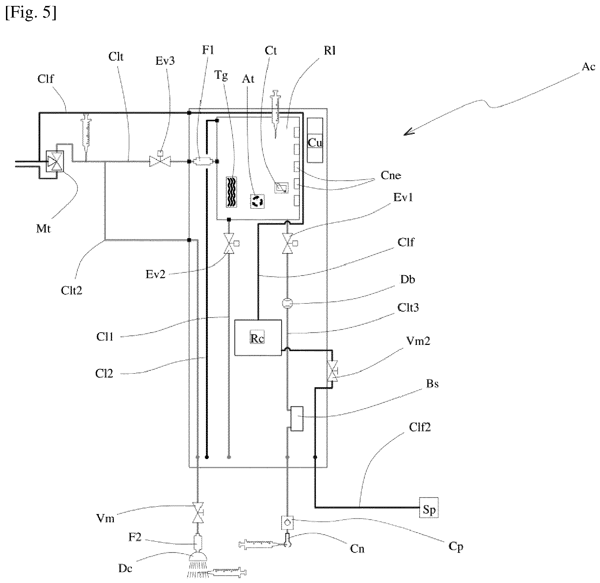

L'armoire de commande Ac contient sur la Fig. 5, un réservoir d'eau de lavage Rl, un calculateur Cu, ainsi que différents capteurs, électrovannes Ev et d'autres accessoires (représentés pour certains à l'extérieur de l'armoire de commande Ac pour des commodités de compréhension, mais qui sont logiquement montés dans l'armoire de commande Ac), des raccordements électriques et hydrauliques, un réservoir de chasse d'eau Rc.

À l'intérieur du réservoir d'eau de lavage Rl, sont montés :
- un thermoplongeur Tg comprenant une résistance électrique qui peut être alimentée en courant électrique pour réchauffer, le cas échéant, l'eau de lavage contenue dans ledit réservoir d'eau de lavage et la maintenir à une température de consigne,
- un agitateur At comprenant une hélice entraînée à rotation, par l'intermédiaire d'un moteur électrique, pour mélanger l'eau contenue dans le réservoir d'eau de lavage Rl afin de maintenir une température homogène partout dans ledit réservoir d'eau de lavage,
- un capteur thermique Ct conçu pour mesurer la température de l'eau dans ledit réservoir,
- plusieurs capteurs de niveau d'eau Cne conçus pour mesurer des niveaux d'eau dans ledit réservoir d'eau de lavage. Ces accessoires sont raccordés au calculateur Cu. Ces capteurs Cne sont répartis dans la hauteur du réservoir d'eau de lavage Rl pour relever plusieurs niveaux de température dans le réservoir d'eau de lavage Rl.

Les branchements en entrée de l'armoire de commande Ac sont les suivants :
- une canalisation d'eau froide Clf provenant d'un circuit d'alimentation en eau froide pour remplir le réservoir de chasse d'eau Rc,
- une canalisation d'alimentation en eau Clt raccordée au réservoir d'eau de lavage Rl. Cette canalisation d'eau Clt provient préférentiellement d'un robinet mitigeur Mt alimenté en eau froide et en eau chaude, pour remplir le réservoir d'eau de lavage Rl. Le réglage de la température de sortie du robinet mitigeur Mt est telle que la canalisation d'eau Clt transporte de l'eau tiède vers le réservoir d'eau de lavage Rl.
- une canalisation d'eau Clt2 provenant, par l'intermédiaire d'une dérivation, du robinet mitigeur alimenté en eau froide et en eau chaude, pour alimenter une douchette Dc. Cette canalisation d'eau Clt2 provient préférentiellement d'un robinet mitigeur Mt alimenté en eau froide et en eau chaude, pour alimenter la douchette Dc. Le réglage de la température de sortie du robinet mitigeur Mt est telle que la canalisation d'eau Clt2 transporte de l'eau tiède vers la douchette Dc,
- un raccordement électrique, non représenté, pour fournir du courant à l'armoire de commande Ac.

Les branchements en sortie de l'armoire de commande Ac sont les suivants :
- visible sur les Figs. 2 et 5, une canalisation de transport d'eau froide Clf2 relie le réservoir de chasse d'eau Rc et un siphon Sp disposé sous le bassin de lavage Bl pour le laver et, le cas échéant, pour le déboucher. La vidange du réservoir de chasse d'eau Rl permet également de nettoyer la canalisation d'évacuation raccordée sur ledit siphon,
- la canalisation d'eau tiède Clt2 chemine au travers de l'armoire de commande Ac, et ressort pour alimenter la douchette Dc,
- une canalisation d'eau tiède de lavage Clt3 raccordée en point bas du réservoir d'eau de lavage Rl et ressort de l'armoire de commande Ac pour alimenter une canule rectale Cn,
- une canalisation Cl1 en provenance d'un point bas du réservoir d'eau de lavage Rl pour le vidanger, le cas échéant, dans un réseau de collecte des eaux usées,
- une canalisation Cl2 en provenance d'un point haut du réservoir d'eau de lavage Rl pour vidanger un éventuel trop plein dans le réseau de collecte des eaux usées. L'armoire de commande Ac comporte des raccords hydrauliques traversant certaines de ses parois permettant le raccordement des canalisations en entrée et en sortie de ladite armoire de commande vers le divan Dv, ainsi qu'une partie des canalisations. Différentes vannes et électrovannes équipent également l'armoire de commande Ac :
- une vanne à commande manuelle Vm est raccordée sur la canalisation d'eau tiède Clt2 qui alimente la douchette Dc,
- une vanne à commande manuelle Vm2 est raccordée sur la canalisation d'eau froide Clf2, en étant disposée à proximité du bassin de lavage Bl, pour déclencher la vidange du réservoir de la chasse d'eau Rl, dans le siphon Sp. Ces deux vannes Vm et Vm2 sont disposées pour être accessibles par un praticien, utilisateur de la machine 100,
- une électrovanne Ev1 est raccordée sur la canalisation d'eau tiède d'eau de lavage Clt3 reliant le réservoir d'eau de lavage Rl et la canule rectale Cn,
- une électrovanne Ev2 est raccordée sur la canalisation de vidange Cl1 reliant le réservoir d'eau de lavage Rl et le réseau de collecte des eaux usées,
- une électrovanne Ev3 est raccordée sur la canalisation d'eau tiède Clt reliant le robinet mitigeur Mt et le réservoir d'eau de lavage Rl.

Dans l'invention, un débitmètre Db est raccordé sur la canalisation d'eau tiède d'eau de lavage Clt3 qui raccorde le réservoir d'eau de lavage Rl avec la canule rectale Cn pour mesurer le débit d'eau qui circule au travers de ladite canule rectale. Le débitmètre Db est raccordé en aval de l'électrovanne Ev1. L'électrovanne Ev1 est utilisée pour autoriser ou stopper l'écoulement de l'eau de lavage vers la canule Cn. Les électrovannes Ev ainsi que le débitmètre Db sont raccordés au calculateur Cu. Dans l'invention, un premier filtre F1 est raccordé sur la canalisation d'eau tiède Clt. Il est raccordé au plus près du réservoir d'eau de lavage Rl. Ce filtre F1 est un filtre de microfiltration comprenant une membrane en fibres creuses. Son efficacité de rétention des germes est de 7 log₁₀ (99,99999 %) selon la norme internationale ASTM F 838-0. L'eau qui ressort du filtre est bactériologiquement maîtrisée, c'est à dire ne contenant aucune bactérie.

Un second filtre F2, de même conception que le premier, est raccordé sur la canalisation d'eau tiède Clt2. Il est préférablement raccordé en aval de la vanne Vm et au plus près de la douchette Dc.

Le calculateur Cu comprend un processeur, une mémoire, au moins un programme d'ordinateur. Ce ou ces programmes sont stockés dans une mémoire morte, par exemple de type EPROM pour ne pas être modifiables facilement par l'utilisateur final de la machine.

Un programme est conçu pour mettre en œuvre la préparation de la machine 100 de lavage du colon et un second programme est conçu pour que la machine 100 puisse mettre en œuvre le cycle de lavage du colon du patient. Un troisième programme de maintenance permet de laver le circuit hydraulique dédié au lavage du colon du patient, de ladite machine. L'armoire de commande Ac comprend également un panneau de contrôle Pn destiné à mettre en œuvre le fonctionnement de la machine 100 et sur lequel sont présents des boutons de commandes, des voyants, un écran de visualisation ainsi qu'un moniteur vidéo.

Un clapet anti-retour Cp est raccordé sur la canalisation d'eau tiède d'eau de lavage Clt3 acheminant l'eau tiède du réservoir d'eau de lavage Rl vers la canule rectale Cn pour empêcher tout retour d'eau de lavage circulant dans la canalisation d'eau tiède d'eau de lavage Clt3, vers le réservoir d'eau de lavage Rl. Le clapet anti-retour Cp est disposé au plus près de la canule rectale Cn, c'est-à-dire à l'extérieur de l'armoire de commande Ac.

Sur la Fig. 5, un bloc de stérilisation Bs de l'eau par un rayonnement ultraviolet est raccordé sur la canalisation d'eau tiède d'eau de lavage Clt3 acheminant l'eau tiède vers la canule rectale Cn, Il est raccordé en aval du débitmètre Db.

Sur la Fig. 2, le bassin de lavage Bl est raccordé au réseau de collecte des eaux usées, par l'intermédiaire d'une canalisation d'évacuation qui chemine dans l'embase Mb et dont au moins une portion est transparente Pt. Une caméra Cm est installée dans l'embase Mb et elle est orientée vers la portion transparente Pt pour la voir. La caméra Cm est reliée à un moniteur vidéo Mv implanté dans l'armoire de commande Ac, ce qui permet pendant leur fonctionnement à un praticien d'évaluer le niveau de turbidité de l'effluent circulant dans ladite canalisation d'évacuation et qui provient du colon du patient. Un moyen d'éclairage peut être installé pour éclairer la scène captée par la caméra. Un fond blanc peut être disposé à l'opposé de la caméra Cm pour augmenter le contraste de la scène captée.

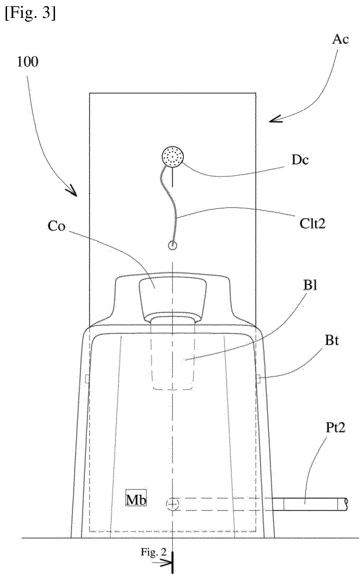

La canalisation d'évacuation au réseau de collecte des eaux usées revient dans l'armoire de commande Ac et ressort de celle-ci pour être raccordée audit réseau de collecte des eaux usées. La portion extérieure de cette canalisation d'évacuation comporte une portion qui est transparente Pt2, visible sur la Fig. 3, ce qui permet au praticien de visualiser par son regard et de contrôler la turbidité des effluents rejetés par le colon du patient.

L'armoire de commande Ac permet de confiner et de protéger dans le volume de ladite armoire de commande les différents accessoires qui viennent d'être présentés c'est-à-dire : le réservoir d'eau de lavage Rl incluant des composants, le calculateur Cu, les électrovannes Ev1, Ev2 et Ev3, le réservoir de chasse d'eau Rc, le débitmètre Db, le bloc de stérilisation Bs, ainsi qu'une partie des canalisations Clf, Clf2, Clt, Clt2, Clt3.

Le divan Dv peut être équipé d'une paire de barrières de sécurité, non représentées, conçues et disposées respectivement de chaque côté du divan Dv afin que le patient installé sur le divan ne puisse tomber à l'extérieur de celui-ci. Chaque barrière de sécurité comprend un cadre baraudé monté de manière articulée sur un côté longitudinal du divan Dv.

Le fonctionnement de la machine 100 se présente de la manière suivante :
On prépare dans un premier temps la machine 100. Le praticien allume la machine à l'aide d'un bouton de commande, puis choisit le programme "préparation" dans le menu proposé sur l'écran de visualisation ou par l'intermédiaire d'un bouton dédié.

Le praticien vérifie la température de consigne du robinet mitigeur Mt qui doit être réglée sur 38 °C.

Le calculateur Cu commande la fermeture des électrovannes Ev1, Ev2, ainsi que l'ouverture de l'électrovanne Ev3, si bien que de l'eau tiède est admise dans le réservoir d'eau de lavage Rl. L'eau admise est filtrée au travers du filtre Fl. Les capteurs de niveau d'eau Cne renseignent le calculateur Cu sur le niveau de remplissage du réservoir d'eau de lavage Rl. Des diodes présentes sur le tableau de commande et raccordées aux capteurs Cne indiquent au praticien le niveau de remplissage du réservoir d'eau de lavage Rl. Dès qu'il est plein, l'électrovanne Ev3 est fermée par le calculateur Cu. Le programme "préparation" est terminé. Le réservoir d'eau de lavage Rl contient 31 litres d'eau bactériologiquement maîtrisée, et ce volume correspond à celui qui est nécessaire pour laver le colon d'un patient adulte. Le thermoplongeur Tg maintient la température de l'eau dans une plage de température de consigne comprise entre 37 °C et 39 °C. Le capteur thermique Ct mesure la température de l'eau contenue dans le réservoir de lavage Rl et renseigne le calculateur Cu. L'agitateur At brasse l'eau. Un voyant s'allume et avertit le praticien que la machine 100 est prête à fonctionner.

On demande au patient de s'allonger sur le divan. Il dispose son anus au-dessus du bassin de lavage Bl. Le praticien introduit la canule rectale Cn dans l'anus du patient. Le praticien peut alors sélectionner le programme "Cycle lavage" sur l'écran de visualisation ou par l'intermédiaire d'un bouton dédié. Le calculateur Cu commande l'ouverture de l'électrovanne Ev1.

Le réservoir d'eau de lavage Rl est disposé à un mètre au-dessus du niveau du bassin de lavage Bl. La tolérance de cette hauteur est de plus ou moins cinquante centimètres.

L'eau stérile contenue dans le réservoir d'eau de lavage Rl s'écoule alors sous l'effet de la gravité dans le colon du patient et pénètre de plus en plus loin, jusqu'au caecum. Le patient contrôle ses sphincters pour que l'eau puisse ressortir de son anus et être rejetée dans le bassin de lavage Bl. Le débitmètre Db mesure la vitesse d'écoulement de l'eau au travers de la canule rectale Cn et renseigne le calculateur Cu.

Les diodes présentes sur le tableau de commande et en relation avec les capteurs Cne indiquent au praticien le niveau de vidange du réservoir d'eau de lavage Rl.

Si le capteur thermique Ct mesure une température supérieure à 39 °C, le calculateur Cu commande immédiatement alors la fermeture de l'électrovanne Ev1. Cette information dénote un dysfonctionnement de la machine. Une alarme est activée, par exemple par l'intermédiaire d'un voyant ou d'un son émis, par exemple par un avertisseur sonore. La sécurité du patient est préservée.

Si le débit mesuré par le débitmètre Db devient nul, le calculateur Cu commande alors la fermeture de l'électrovanne Ev1. Une alarme est activée, par exemple par l'intermédiaire d'un voyant ou d'un son émis, par exemple par un avertisseur sonore. Le praticien doit alors intervenir pour repositionner convenablement la canule rectale Cn ou pour retirer un amalgame de matières fécales susceptibles de gêner la circulation des eaux de lavage.

Après cette intervention, le praticien doit réactiver le programme "Cycle lavage" en utilisant le menu proposé sur l'écran de visualisation ou par l'intermédiaire d'un bouton dédié.

Le praticien peut utiliser la douchette Dc en actionnant la vanne manuelle Vm pour nettoyer le patient.

Le praticien peut également utiliser le moniteur vidéo pour contrôler la turbidité des effluents rejetés par le patient.

Lorsque le réservoir d'eau de lavage Rl est vide, une alarme visuelle ou sonore, raccordée au calculateur Cu est émise. Le programme "cycle de lavage" est terminé. À tout moment le patient peut interrompe de lui-même le cycle de lavage. Il peut utiliser, en référence à la Fig. 1, un bouton d'arrêt Bt disposé à portée de main, sur au moins un coté de l'embase Mb et qui est relié au calculateur Cu. Celui-ci commande immédiatement la fermeture de l'électrovanne Ev1 si bien que l'écoulement de l'eau de lavage dans son rectum est interrompu. Une alarme est activée, par exemple par l'intermédiaire d'un voyant ou d'un son émis, par exemple par un avertisseur sonore. Le praticien intervient alors pour comprendre et aider le patient.

Le praticien débranche le patient et utilise la douchette Dc pour nettoyer le patient qui peut ensuite quitter le divan Dv. L'eau fournie par la douchette Dc est également stérile, c'est-à-dire bactériologiquement maîtrisée, du fait de la présence du second filtre F2 en amont.

Le praticien peut ensuite actionner la vanne à commande manuelle Vm2 ce qui provoque la vidange de l'eau contenue dans le réservoir de chasse d'eau Rc, dans le siphon Sp raccordé sous le bassin de lavage Bl. Le réservoir de chasse d'eau Rc se remplit à nouveau.

Sur la Fig. 2, la douchette Dc est raccordée, par l'intermédiaire d'une portion de sa canalisation Clt2 à un raccordement traversant une paroi de l'armoire de commande Ac. Un support pour la douchette Dc est fixé sur ladite paroi.

La canule rectale Cn est raccordée, par l'intermédiaire d'une portion de sa canalisation d'eau de lavage Clt3 à un raccordement monté dans le bassin de lavage Bl.

La maintenance de la machine 100 est avantageusement mise en œuvre de la manière suivante afin qu'elle puisse continuer à fournir régulièrement une eau de lavage stérile. La maintenance de la machine est proposée périodiquement par un programme " désinfection maintenance" mis en œuvre dans le calculateur Cu. La périodicité peut être quotidienne, hebdomadaire ou mensuelle. Le protocole suivant de désinfection est mis en œuvre :
L'opérateur de maintenance verse 30 ml d'eau de Javel^{®} dans le réservoir d'eau de lavage Rl, puis démarre le programme "désinfection maintenance". L'électrovanne Ev1 est ouverte par le calculateur Cu et l'eau de lavage désinfecte, le réservoir d'eau de lavage Rl (ainsi que les composants qu'il contient), la canalisation Clt3,

l'électrovanne Ev1, le débitmètre Db, le bloc de stérilisation Bs, le clapet anti-retour Cp, ainsi que la canule rectale Cn. Lorsque le réservoir de lavage Rl est vide, le programme "désinfection maintenance" lance ensuite un cycle de rinçage.

L'électrovanne Ev1 est fermée, puis le réservoir de lavage Rl est rempli à nouveau.

L'électrovanne Ev1 s'ouvre à nouveau et l'eau de rinçage rince le réservoir d'eau de lavage Rl (ainsi que les composants qu'il contient), la canalisation Clt3,

l'électrovanne Ev1, le débitmètre Db, le bloc de stérilisation Bs, le clapet anti-retour Cp, ainsi que la canule rectale Cn. La machine est prête pour une nouvelle utilisation.

Des prélèvements sont réalisés à intervalles réguliers pour déceler la présence de germes dans la machine 100. Les prélèvements sont réalisés dans la canalisation Clt, à la sortie du robinet mitigeur Mt, dans le réservoir d'eau de lavage Rl, dans la canule rectale Cn, à la sortie de la douchette Dc. Ces prélèvements sont indiqués par la présence de seringues sur la Fig. 5.

Si ces prélèvements sont positifs, c'est-à-dire lorsque la mesure de la flore microbienne est supérieure à la norme en vigueur, une maintenance d'un second niveau de la machine est entreprise et le protocole de décontamination suivant peut être mis en œuvre directement par l'opérateur :
L'opérateur de maintenance verse 30 ml d'eau de Javel^{®} ainsi qu'un détergent dans le réservoir d'eau de lavage Rl, puis démarre le programme " désinfection maintenance " et un cycle de décontamination est lancé, suivi d'un cycle de rinçage, semblable à celui décrit précédemment. La machine est prête pour une nouvelle utilisation.

Pour obtenir une décontamination encore plus efficace, et dans un mode opératoire d'un troisième niveau, le protocole de décontamination suivant est mis en œuvre : L'opérateur de maintenance verse 100 ml d'eau de Javel^{®} ainsi qu'un détergent dans le réservoir d'eau de lavage Rl, puis démarre le programme "désinfection maintenance" et un cycle de décontamination est lancé, suivi d'un cycle de rinçage, semblable à celui décrit précédemment.

Le circuit est rincé. La machine est prête pour une nouvelle utilisation.

L'électrovanne Ev2 sert à vidanger l'eau éventuellement contenue dans le réservoir d'eau de lavage Rl pour l'évacuer dans le réseau de collecte des eaux usées. Son fonctionnement est mis en œuvre dans le menu proposé sur l'écran de visualisation ou par l'intermédiaire d'un bouton de commande dédié.

La machine 100 de l'invention est conçue pour mettre en œuvre, de manière autonome, le lavage du colon d'un patient.

Son fonctionnement est sûr et répond à une conception et à l'intégration de dispositifs de contrôle qui assurent une sécurité optimale pour une utilisation en milieu hospitalier public et privé.

Elle peut être utilisée par des personnels formés et dans des conditions de contrôle, de sécurité et d'hygiène spécifiques.

## Revendications

1. Machine (100) conçue pour mettre en œuvre le lavage du colon d'un patient, comprenant, un divan (Dv) incluant un bassin de lavage (Bl), un réservoir d'eau de lavage (Rl) disposé plus haut que le bassin de lavage (Bl), dans la position d'utilisation de ladite machine, une canalisation (Clt) d'alimentation en eau raccordée au réservoir d'eau de lavage (Rl), une canule rectale (Cn) raccordée au réservoir d'eau de lavage (Rl), par l'intermédiaire d'une canalisation d'eau de lavage (Clt3), un débitmètre (Db) étant raccordé sur la canalisation d'eau de lavage (Clt3), **caractérisée en ce qu'**une électrovanne (Ev1) est raccordée sur la canalisation d'eau de lavage (Clt3) qui raccorde le réservoir d'eau de lavage (Rl) avec la canule rectale (Cn), la machine (100) comprenant une armoire de commande (Ac) incluant, un calculateur (Cu), un programme mis en œuvre dans le calculateur (Cu), l'électrovanne (Ev1) et le débitmètre (Db) étant raccordés au calculateur (Cu) et **en ce que** le programme contient des instructions pour couper le débit d'eau circulant dans l'électrovanne (Ev1), quand le débit d'eau circulant dans le débitmètre (Db) devient nul, et activer une alarme visuelle ou sonore.

2. Machine (100) selon la revendication 1, **caractérisée en ce qu'**elle comprend un capteur thermique (Ct) monté dans le réservoir d'eau de lavage (Rl), le capteur thermique (Ct) étant raccordés au calculateur (Cu) et **en ce que** le programme contient des instructions pour couper le débit d'eau circulant dans l'électrovanne (Ev1), quand la température mesurée par le capteur thermique est supérieure à 39°C, et activer une alarme visuelle ou sonore.

3. Machine (100) selon l'une quelconque des revendications précédentes, **caractérisée en ce qu'**un filtre (F1) est raccordé sur la canalisation (Clt) d'alimentation en eau, et **en ce que** ledit filtre est un filtre de microfiltration comprenant une membrane en fibres creuses.

4. Machine (100) selon l'une quelconque des revendications précédentes, **caractérisée en ce qu'**elle comprend une douchette (Dc) raccordée à une seconde canalisation d'alimentation en eau (Clt2), et **en ce qu'**un second filtre (F2) est raccordé sur ladite canalisation (Clt2) d'alimentation en eau, le second filtre (F2) étant un filtre de microfiltration comprenant une membrane en fibres creuses.

5. Machine (100) selon l'une quelconque des revendications précédentes, **caractérisée en ce que** le réservoir d'eau de lavage (Rl) contient un thermoplongeur (Tg), un agitateur (At), et qui sont raccordés au calculateur (Cu).

6. Machine (100) selon l'une quelconque des revendications précédentes, **caractérisée en ce que** plusieurs capteurs de niveau d'eau (Cne) sont montés dans le réservoir d'eau de lavage (Rl) pour mesurer des niveaux d'eau dans le réservoir d'eau de lavage (Rl), lesdits capteurs étant raccordés au calculateur (Cu).

7. Machine (100) selon l'une quelconque des revendications précédentes, **caractérisée en ce que** l'armoire de commande (Ac) contient un réservoir de chasse d'eau (Rc) raccordé à un siphon (Sp) raccordé sous le bassin de lavage (Bl), par l'intermédiaire d'une vanne à commande manuelle (Vm2).

8. Machine (100) selon l'une quelconque des revendications précédentes, **caractérisée en ce que** le bassin de lavage (Bl) est raccordé à un réseau de collecte des eaux usées, par l'intermédiaire d'une canalisation d'évacuation et dont au moins une portion (Pt) est transparente, une caméra (Cm) étant orientée vers la portion transparente (Pt) pour la voir, la caméra (Cm) étant reliée à un moniteur vidéo (Mv) implanté dans l'armoire de commande (Ac).
